# EUROPEAN PATENT SPECIFICATION

(11) **EP 3 431 127 B1**
(45) Date of publication and mention of the grant of the patent: **20.01.2021**
(21) Application number: 17181765.3
(22) Date of filing: 18.07.2017
(51) Int. Cl.: A61M 15/00

(54) **METERED DOSE INHALER**
DOSIERINHALATOR
INHALATEUR DE DOSE MESURÉE

(43) Date of publication of application: 23.01.2019
(73) Proprietor: Presspart GmbH & Co. KG, 34431 Marsberg (DE)
(72) Inventor: Jung, Benjamin, 50674 Köln (DE); Seiler, Matthias, 40627 Düsseldorf (DE); Schmelzer, Hans-Peter, 40667 Meerbusch (DE); Hemy, Julian, Warrington, Cheshire WA4 3JA (GB); Sule, Ameet, 401107 Maharashtra (IN); Turner, Richard, Simonstone, Lancashire BB12 7ST (GB); Torres, Victor, 08197 Valldoreix, Barcelona (ES)
(74) Representative: Uexküll & Stolberg

(56) References cited:
- EP-A1- 3 363 485
- WO-A1-00/64517
- WO-A1-2016/030844
- WO-A2-2005/009325
- DE-U1-202017 101 591
- US-A- 5 020 527

## Description

### TECHNICAL FIELD

The present invention relates to metered dose inhalers for dispensing aerosol doses. The inhalers comprise an actuator housing in which an aerosol container with an activation valve is inserted. The container is configured to move in a longitudinal direction from a rest position to an activation position in which the valve is depressed against a bottom portion of the actuator housing such that an aerosol dose is released. A triggering unit comprises at least a first trigger member, which is configured to interact with the container moving in the longitudinal direction. The at least first trigger member triggers at least a first switch of an electronic unit when the container reaches a first longitudinal position during its movement from the rest position to the activation position. A counting circuit is configured to receive a signal from the at least first switch indicating at least a first time when the first switch is triggered by the first trigger member.

### BACKGROUND OF THE INVENTION

Metered dose inhalers (MDIs) are medication delivery devices which deliver a pharmaceutical formulation including one or more pharmaceutically active compounds to a human or other mammalian patient. Typically, the pharmaceutical formulation is delivered by the MDIs in unit doses in the form of an aerosol. Each actuation of the MDIs delivers one unit dose. The unit dose is expelled by the MDIs and is taken into the body of the patient on inhalation, via the nose or mouth. The pharmaceutical formulation is delivered to or via the respiratory tracts, notably to the lungs, of the patient on inhalation. Metered dose inhalers are typically used for the treatment of respiratory infections and disorders including respiratory tract infections, obstructive lung disease, inflammatory lung disease and chronic obstructive pulmonary disease. Asthma treatment is a particularly common use of MDIs.

Metered dose inhalers typically comprise a dose counter. Dose counters allow counting of the doses which are released from the container such that the patient inhaling the doses is always aware of the doses being left in the container. These dose counters may operate either mechanically or electronically.

There has been a recent development toward use of electronic dose counters, as they allow the implementation of additional functions such as monitoring functions or evaluation functions. Based on these additional functions a physician or the patient may for example monitor the frequency of dispensed doses and the point of time when these doses have been dispensed.

WO 2016/030844 A1 describes a metered dose inhaler with an actuation housing in which two separate trigger members are actuated by the container in two different positions of the container. The first trigger member is actuated upon displacement of the container from the rest position to a first position, thereby indicating that the user intends to dispense a dose of the aerosol. The container is then moved from the first position to a second position in which the valve is opened and the aerosol is dispensed. When the aerosol is dispensed, the second trigger member is actuated. Upon actuation the first and second trigger members trigger first and second switches of a circuit assembly. A counting circuit receives signals from the first and second switches when being triggered and counts the number of dispensed doses.

Co-pending European Patent Application No. 17156995.7 also discloses a metered dose inhaler with an actuation housing and an electronic dose counter. Upon movement of a container from a rest position to an activation position in which an aerosol dose is released a single trigger member is actuated. Upon actuation the trigger member interacts with a switch generating an electrical signal that is transmitted to a processing unit. The processing unit processes the electrical signal.

In the field of MDIs, it is desired to provide reliable and cost-effective products. As the electronic dose counter comprises several electronic components it forms a costly component of a metered dose inhaler. In order to reduce costs, it would be desirable to re-use the electronic dose counter when for example the actuator housing is damaged and has to be replaced.

Reusable electronic dose counters which can be attached and detached from a metered dose inhaler are known. A re-usable electronic dose counter is commercially available under the trade name HeroTracker™ from Cohero Health. The HeroTracker™ is affixed to the top of a metered dose inhaler via straps such that the upper part of the metered dose inhaler as well as the container are covered. A manual depression of the container in order to release a dose of medicament contained therein is sensed by the trigger sensor and recorded and/or wirelessly transmitted to other devices. Moreover, as the HeroTracker™ comprises several straps and electronic components a metered dose inhaler having the HeroTracker™ installed thereon is rather bulky.

It is an object of the present invention to provide a cost effective and highly reliable metered dose inhaler which is convenient in terms of use.

DE 20 2017101 591 relates to a device for oral or nasal administration of a pharmaceutical formulation to the respiratory tract of a patient.

EP 3 363 485 relates to a metered dose inhaler for dispensing aerosol doses and is prior at according to Article 54(3) EPC.

### SUMMARY OF THE INVENTION

This object is achieved by a metered dose inhaler comprising the features of claim 1, and an add-on device comprising the features of claim 13. Preferred embodiments are set out in the dependent claims.

According to the present invention the electronic unit is detachable from the actuator housing independently from the triggering unit. Moreover, the electronic unit is configured to be re-usable in combination with another e.g. identical or similar metered dose inhaler.

The general function of the triggering unit comprising two trigger members is disclosed in international patent application WO 2016/030844 A1. Moreover, the general function of the triggering unit comprising one single trigger member is disclosed in European Patent Application No. 17156995.7. The triggering unit together with the electronic unit have proven to provide reliable detection of actuation of the medicament container and thus reliable counting of the released aerosol doses. In particular, it is advantageous to install the triggering unit in the interior of the actuator housing as this avoids unintended dose counts for example when the metered dose inhaler is dropped on the floor.

As the electronic unit is detachable from the actuator housing independently from the triggering unit and is re-usable, the metered dose inhaler can be provided very cost effectively. The metered dose inhaler may be distributed with or without the electronic unit. Moreover, the electronic unit may be available separately. Thus, a patient may in case of any damage to the actuator housing or the electronic unit, purchase only the damaged part and re-use the rest of the metered dose inhaler. This allows replacement of the actuator housing or the electronic unit for the patient.

Preferably the metered dose inhaler is configured to be used without an electronic unit. In this case a dummy part may be attached to the actuator housing instead of the electronic unit. The dummy part is configured to seal any wholes or openings in the actuator housing which would be closed when the electronic unit is attached. Thus a comparable airflow during inhalation is guaranteed as if the electronic unit is installed. In case the electronic unit is detached from the actuator housing the metered dose inhaler does not provide an electronic dose counting function. However, the metered dose inhaler may comprise a mechanical dose counter such that a counting of released doses is possible, despite the electronic unit being detached. Preferably the mechanical dose counter is installed in the longitudinal direction between the container and the bottom portion of the actuator housing.

In connection with the present invention the term "detachable" is understood in such a way that the electronic unit may be non-destructively separated from the actuator housing. The electronic unit as well as the actuator housing are specifically designed in order to allow such detachment. Detachment of the electronic unit from the actuator housing does not damage the electronic unit or the actuator housing in any way and thus allows a proper function of the electronic unit installed in another metered dose inhaler.

In connection with the present invention the term "counting circuit" is understood as an electronic counting unit configured to receive signals from the at least first switch indicating at least a first time when the at least first switch is triggered by the first trigger member, and to count the dispensed or remaining doses left in the container in dependence of the received signal(s).

According to an embodiment of the invention, the electronic unit is positioned at the rear side of the actuation housing. Preferably, the electronic unit is located in proximity to a first open end of the actuator housing sized and arranged to receive the aerosol container. This allows a positioning of the activation valve of the container close to a second open end of the actuator housing through which an aerosol dose released from the container is dispensed. Such close positioning reduces the deposition of released aerosol at the inner walls close to the second open end of the metered dose inhaler which is preferably designed as a mouthpiece. Moreover, the positioning of the electronic unit close to the first open end at the top of the actuator housing leads to a handy design of the metered dose inhaler.

In a further embodiment of the present invention the metered dose inhaler comprises a carrier for carrying and fixing the electronic unit wherein the carrier has attachment members for being attached to the actuator housing. The carrier is constructed to protect the electronic unit such that upon detachment of the electronic unit from the actuator housing and during re-attachment to the actuator housing of another metered dose inhaler, the electronic unit is not damaged. Preferably, the electronic unit comprises a substrate in the form of a printed circuit board which is clipped or fixed to the carrier by any known clipping or fixing method. Optionally the attachment members are positioned on the carrier whereas the actuation housing comprises corresponding receiving members for receiving the attachment members or vice versa. Preferably the attachment members as well as the corresponding receiving members are interrelated in such a way that only electronic units and/or actuator housings distributed by the applicant of the present invention may be attached. The attachment members as well as the corresponding receiving members can also be specific to a medicine or a group of medicines. Thus, misuse of the electronic unit with metered dose inhalers, for example of other companies, can be avoided.

Preferably, the electronic unit comprises an additional switch which is triggered when the electronic unit is attached to a metered dose inhaler. Alternatively, the at least first switch is triggered once by the at least first trigger member when the electronic unit is attached to a metered dose inhaler. The electronic unit may recognize this triggering as an indication that it was attached to a metered dose inhaler. Optionally, the container or the actuator housing comprises an identification mark such as a RFID tag or a QR-code identifying for example the medicament contained in the container or the manufacturer of the medicament. Alternatively or in addition thereto, the container or the actuator housing may comprise a numeric or alphanumeric number such as a production number. Upon triggering the at least first or additional switch during attachment, the electronic unit automatically reads the information stored on the RFID tag or requests a user to read/scan the QR-code or the RFID tag via a mobile device such as a smartphone. Subsequently the mobile device may wirelessly transmits the read/scanned identification mark to the electronic unit or vice versa. This requires a wireless connection between the electronic unit and the mobile device. Electronic units configured to establish such a wireless connection are well known. In case the identification mark is known to the electronic unit the electronic might start working. In case the identification mark is unknown the electronic unit might shut off. Furthermore, in case the metered dose inhaler comprises a mechanical dose counter in addition, the electronic unit or the mobile device might request the patient upon attachment to read out the number of mechanically count doses and to enter the number of count doses into the mobile device. Alternatively, the mobile phone could be used to read the mechanical counter. Subsequently the mobile device wirelessly transmits the data to the electronic unit which is, based on this data, able to for example display the number of remaining doses in the container. Moreover, when the identification mark of the container or the actuator housing is known the electronic unit may for example not only count the number of doses released from the container but may also store the number such as the production number of the container in use. Alternatively or additionally the mobile device displays the number of remaining doses and may store additional information such as the production number of the container.

Optionally, the electronic unit may only be attachable to a metered dose inhaler by a specialized person such as a physician or a pharmacist, in order to avoid damages to the electronic unit or the metered dose inhaler during attachment. This may be realized by the electronic unit requesting to enter a password only known to the specialized person into a mobile device upon attachment of the electronic unit to the actuator housing.

According to an embodiment of the invention the carrier comprises a release mechanism configured to release the attachment members upon manual actuation thereby allowing detachment of the carrier together with the electronic unit from the actuator housing. Such a release mechanism allows easy detachment of the electronic unit from the actuator housing. At the same time, this release mechanism guarantees a secure fixation of the electronic unit to the actuator housing as manual actuation is necessary to allow detachment of the electronic unit. In connection with the present invention, the term "manual actuation" has to be understood in such a way that the patient or pharmacist has to apply force to a certain level, for example onto opposing walls of the carrier, such that the attachment members of the carrier are detached and the carrier is released from the actuator housing. Preferably the force may be applied by two fingers of one hand pressing onto the opposing walls.

In a further embodiment of the invention the carrier is formed as an add-on housing, having an opening on one side facing the actuator housing in order to allow interaction between the at least first trigger member and the at least first switch. Preferably, the add-on housing surrounds the electronic unit except for the opening through which the electronic unit interacts with the trigger member. This provides proper protection of the electronic unit during attachment to the actuator housing as well as when being detached and re-installed to another metered dose inhaler. Moreover, the electronic unit is protected during use of the metered dose inhaler. Alternatively the add-on housing comprises a flexible area in order to allow interaction between the at least first trigger member and the at least first switch such that upon actuation the at least first trigger member deforms the flexible area of the add-on housing such that the at least first switch is triggered.

In a still further embodiment of the invention the add-on housing is connected with the actuator housing via a snap-in connection, wherein the attachment members are configured as snap-in members. The add-on housing may for example comprise one or more snap-in tongues which engage with corresponding openings in the actuator housing thereby forming a form-fitting connection of the add-on housing and the actuator housing.

Also preferred is an embodiment according to which the at least first trigger member extends from the rear side of the actuator housing towards the second end of the actuator housing, wherein the at least first trigger member is configured to at least partially deform the rear side of the actuator housing in order to trigger the first switch. This allows the fabrication of the actuator housing together with the trigger members in one piece. Preferably, the actuator housing is fabricated by injection molding. Upon movement of the container in the longitudinal direction from the rest position to the activation position the at least first trigger member may be moved lateral thereto which causes an at least partial deformation of the rear side of the actuator housing in particular a bending of the rear side of the actuator housing. The deformation of the rear side of the actuator housing is chosen in such a way that the switches of the electronic unit are reliably triggered. In case it is intended to use the metered dose inhaler without the electronic unit, a dummy part designed as a plate may be attached, for example clipped, to the rear side of the actuator housing instead of the electronic unit. The plate is of similar shape as the rear side of the actuator housing and seals any wholes or openings in the actuator housing which would be closed when the electronic unit is attached.

Alternatively, the at least first trigger member optionally extends from a rear insert inserted in the rear side of the actuator housing towards the second end of the actuator housing, wherein the at least first trigger member is configured to at least partially deform the rear insert of the actuator housing in order to trigger the first switch. For example, the rear insert containing the first trigger member may be formed as a plane wall and may be slid into corresponding guiding elements positioned on the actuator housing.

However, the deformation of the rears side of the actuator housing is an elastic deformation such that the rears side of the actuator housing takes its original shape when the container moves back to the rest position.

In particular preferred is an embodiment according to which the carrier is formed as a frame to which the electronic unit is fixed wherein the rear side of the actuator housing forms a receiving portion in proximity to the first end of the actuator housing, the receiving porting being sized and arranged to receive the frame together with the electronic unit such that the electronic unit is positioned between the rear side of the actuator housing and the container. Preferably, the receiving portion comprises a top opening towards a top of the actuator housing. Preferably, the frame together with the electronic unit is slid from above into the receiving portion which may comprise corresponding guiding elements. The size of the receiving portion is chosen such that a proper handling of the metered dose inhaler by the patient, in particular a proper grip of the inhaler during inhalation, is still guaranteed. The frame may for example also comprise a top cover which covers the top opening of the receiving portion at the top of the actuator housing. By inserting the electronic unit into the receiving portion, the electronic unit is properly protected from mechanical forces acting on the actuator housing. The electronic unit is preferably fully surrounded by the actuator housing and/or an additional top cover fixed to the frame. In connection with the present invention the term "frame" is to be understood as forming a connecting element in between the electronic unit and the receiving portion of the actuator housing, wherein the frame leaves the electronic unit uncovered. However, in connection with the present invention the term "frame" may also be understood as a part which fully covers the electronic unit, such that the frame surrounds the electronic unit and forms an insert which is received by the receiving portion of the actuator housing. In this case the frame comprises an opening in order to allow interaction between the at least first trigger member and the at least first switch. Alternatively, the frame may comprise a flexible area which is flexibly deformed by the trigger member upon actuation. In case the frame forms an insert the insert as well as the receiving portion may be interrelated such that only inserts having a geometric form corresponding to the geometric form of the receiving portion may be inserted into the receiving portion of the actuator housing. Preferably, only the inserts and/or the actuator housings fabricated by the same manufacturer, preferably the applicant of the present invention, are interrelated as described above. The insert as well as the corresponding receiving portion can also be specific to a medicine or a group of medicines. Thus, misuse of the electronic unit with metered dose inhalers, for example of other companies, can be avoided.

Moreover, positioning of the electronic unit between the rear side of the actuator housing and the container allows easy access and thus easy detachment of the electronic unit from the actuator housing. Preferably, the attachment members as well as the corresponding receiving members are interrelated in such a way that only electronic units and/or actuator housings distributed by the applicant of the present invention may be attached. The attachment members as well as the corresponding receiving members can also be specific to a medicine or a group of medicines. Thus, misuse of the electronic unit with metered dose inhalers, for example of other companies, can be avoided.

According to one embodiment of the invention the frame is connected with the actuator housing via a snap-in connection, wherein the attachment members are configured as snap-in members. Preferably, the snap-in connection is designed as a form-fitting connection wherein the frame comprises for example snap-in tongues which engage with corresponding openings in the actuator housing or vice versa such that a form-fitting connection in between the frame and the actuator housing is provided.

Also preferred is an embodiment according to which the actuator housing comprises a middle wall positioned between the first end of the actuator housing and the receiving portion, wherein the at least first trigger member extends from the middle wall towards the second end of the actuator housing and is configured to at least partially deform the middle wall of the actuator housing in order to trigger the first switch. In case it is intended to use the metered dose inhaler without the electronic unit, a dummy part in the form of a plate may be attached, for example clipped, to the middle wall instead of the electronic unit. The plate is of similar shape as the middle wall and seals any wholes or openings in middle wall and/or the actuator housing which would be closed when the electronic unit is attached. The dummy part may also be designed as an insert. The outer walls of the insert may have the same shape as the receiving portion such that upon insertion of the insert into the receiving portion the insert fully fills the receiving portion.

The middle wall may be produced as part of the actuator housing in one piece preferably by injection molding. Alternatively, the middle wall may be produced as a separate component being inserted into the actuator housing. The middle wall does not only provide a fixation for the first trigger member but also protects the electronic unit when the container is inserted into the first open end of the actuator housing. The deformation of the middle wall upon interaction between the container and the trigger member is chosen such that the switch of the electronic unit is triggered. Nevertheless, the deformation of the middle wall is an elastic deformation such that the middle wall takes its original shape when the container moves back to the rest position.

According to a further embodiment of the invention, the longitudinal position of the container is either the activation position of the container or a position of the container in which the valve abuts the bottom portion of the actuator housing in order to be depressed. However, the triggering of the switch is indicative of a release of an aerosol dose from the container. Moreover, in case of one single trigger member, the location of the first longitudinal position is chosen at a position which effectively prevents unwanted actuation of the switch when the metered dose inhaler falls on the ground. Thus, a reliable indication of the administration of the patient's medication is ensured. When the first longitudinal position is the activation position, the switch is triggered when an aerosol dose is released by the valve. However, it might be desired to trigger the first switch when the container reaches a position located in longitudinal direction before the activation position. Such a position allows the compensation of a reduction of elasticity in the components of the triggering unit and thus ensures reliability of the metered dose inhaler during its life cycle. Such a position may be chosen as a position of the container in which the valve abuts the bottom portion of the actuator housing in order to be depressed.

In an embodiment of the invention a triggering unit has at least a first and a second trigger member thereon and that the substrate of the electronic unit has at least a first and a second switch thereon, wherein the second switch is configured to interact with the second trigger member when the container moves from the rest position to the activation position such that the second trigger member triggers the second switch when the container reaches a second longitudinal position that is different from the first longitudinal position during movement of the container from the rest position to the activation position and, wherein the counting circuit is configured to receive a signal from the second switch indicating a second time when the second switch is triggered by the second trigger member. A general function of a metered dose inhaler comprising a first and a second trigger member and corresponding first and second switches is described in document WO 2016/030844 A1.

The use of two trigger members may improve the reliability as the speed of the container being depressed from the rest position to the actuation position may be detected.

The invention also relates to an add-on device for being attached to an actuator housing of a metered dose inhaler in particular as described herein. Properties and constructive features of the add-on device described herein in connection with the metered dose inhaler according to the invention also account for the inventive add-on device and further embodiments thereof.

The add-on device comprises an electronic unit having a substrate with at least a first switch thereon, the first switch being configured to interact with a first trigger member of the metered dose inhaler when a container received in an actuator housing of the metered dose inhaler moves from a rest position to an activation position such that the first trigger member triggers the first switch when the container reaches a longitudinal position during movement of the container from the rest position to the activation position. The add-on device further comprises a counting circuit that is configured to receive a signal from the first switch indicating at least a first time when the first switch is triggered by the first trigger member. The counting circuit is arranged on the substrate of the electronic unit. Moreover, the add-on device comprises a carrier for carrying the electronic unit. The carrier comprises attachment members for attaching the add-on device to the actuator housing of a metered dose inhaler in such a way that the first switch is triggerable by the trigger member of the metered dose inhaler or an additional switch is triggered.

The add-on device may be sold separately or in a combination with a metered dose inhaler. A separate distribution allows the patient to replace only the add-on device when the actuator housing and the rest of the metered dose inhaler is still in a proper shape but for example only the electronic unit is damaged.

In an embodiment, the carrier is formed as an add-on housing having an opening on a side of the add-on housing configured to allow interaction between the at least first trigger member and the at least first switch. The add-on housing provides a proper protection of the electronic unit, in particular during attachment to an actuator housing as the electronic unit is fully covered by housing walls of the add-on housing except the opening. Alternatively the add-on housing comprises a flexible area in order to allow interaction between the at least first trigger member and the at least first switch such that upon actuation the at least first trigger member deforms the flexible area of the add-on housing such that the at least first switch is triggered.

According to a further embodiment of the invention the carrier is formed as a frame to which the electronic unit is fixed, wherein the frame is configured to be received in a receiving portion formed by a rear side of the actuation housing, such that the electronic unit is positioned between the rear side of the actuator housing and the container. Preferably, the frame also comprises a top cover which closes an opening of the receiving portion at the top of the actuator housing.

In an embodiment of the invention the metered dose inhaler together with the electronic unit may be inserted into a charging station such that the battery of the electronic unit is charged. This increases the lifetime of the battery. Optionally the charging station may be formed to receive the electronic unit or the add-on housing alone in order to charge the battery contained therein. Charging of the battery may be accomplished by using wireless power transmission which is known in the art.

The invention also relates to a metered dose inhaler for dispensing aerosol doses comprising the features of claim 20. Sensing a dose of medicament when being passed to the nozzle block and flowing therethrough is highly reliable. Thus, miscount of doses being released from the container are avoided.

### BRIEF DESCRIPTION OF THE FIGURES

The invention will now be described in connection with two exemplary embodiments shown in the Figures in which:
- Figure 1: shows a sectional view of a metered dose inhaler;
- Figure 2: shows a top view of the metered dose inhaler according to Figure 1;
- Figure 3: shows a sectional view of a second embodiment of a metered dose inhaler and
- Figure 4: shows a top view of the metered dose inhaler according to Figure 3.

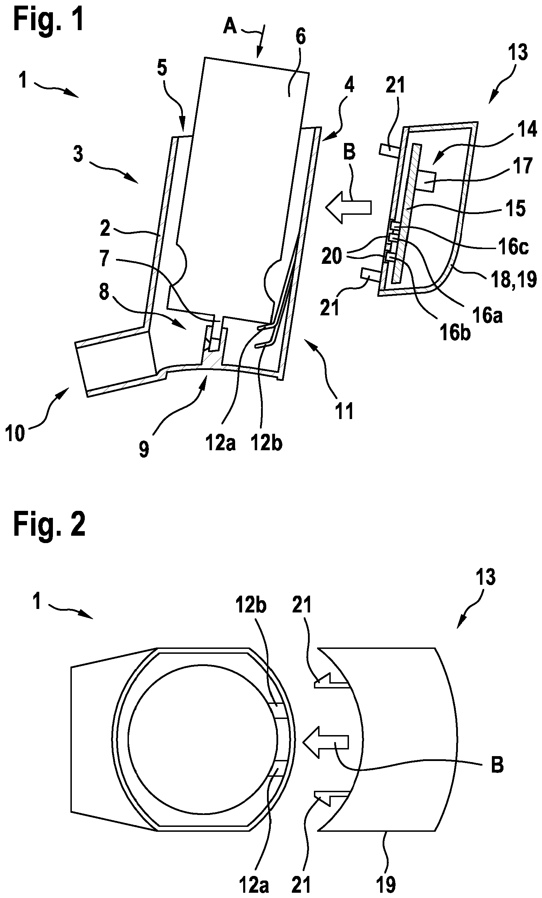

Figures 1 and 2 show a metered dose inhaler (MDI) 1 for dispensing an aerosol dose. The metered dose inhaler 1 comprises an actuator housing 2 with a front side 3 and a rear side 4 opposite the front side. The actuator housing 2 further comprises a first open end 5 which is sized and arranged to receive an aerosol container 6 with an activation valve 7 at an valve end 8 of the container 6. The container 6 is configured to move upon manual actuation by a patient in a longitudinal direction A from a rest position to an activation position in which the valve 7 is depressed against a bottom portion 9 of the actuator housing 2 such that an aerosol dose is released from the container 6.

The actuator housing 2 further comprises a second open end 10 which is formed as a mouthpiece and through which the released aerosol dose is dispensed. The second open end 10 is positioned on the front side 3 of the actuator housing 2. It is sized and arranged to be coupled to the mouth or nasal cavities of a patient (not shown).

The metered dose inhaler 1 further comprises a triggering unit 11 with a first trigger member 12a and a second trigger member 12b thereon. The triggering unit 11 is configured to interact with the container 6 when the container 6 moves in the longitudinal direction A from the rest position to the activation position. The first and second trigger members 12a, 12b extend from the rear side 4 of the actuator housing 2. The first and second trigger members 12a, 12b are configured to at least partially deform the rear side 4 of the actuator housing 2 when being in interaction with the container 6 moving from the rest position to the activation position. The triggering unit 11 is fixed to the actuator housing 2 or is formed integrally with the actuator housing 2.

Furthermore, the metered dose inhaler 1 comprises an add-on device 13 for being attached to an actuator housing 2 of the metered dose inhaler 1. The add-on device 13 comprises an electronic unit 14 having a substrate with a first switch 16a and a second switch 16b thereon. The first and second switches 16a, 16b are configured to interact with the first and second trigger members 12a, 12b when the container 6 moves from the rest position to the activation positions such that the first trigger member 12a triggers the first switch 16a when the container reaches a first longitudinal position and, wherein the second trigger member 12b triggers the second switch 16b when the container 6 reaches a second longitudinal position during movement of the container 6 from the rest position to the activation position. It is noted that the first longitudinal position differs from the second longitudinal position of the container 6.

The add-on device 13 further comprises a counting circuit 17 which is configured to receive a signal from the first and second switches 16a, 16b indicating at least first and second times when the first and second switches 16a, 16b are triggered by the first and second trigger members 12a, 12b, respectively. The counting circuit 17 is arranged on the substrate 15 of the electronic unit 14.

The add-on device 13 further comprises a carrier 18 for carrying and fixing the electronic unit 14. The carrier 18 is formed as an add-on housing 19 having one or two openings 20 on one side facing the actuator housing 20 in order to allow interaction between the first and second trigger members 12a, 12b and the first and second switches 16a, 16b.

The add-on housing 19 has attachment members which are formed as snap-in members 21 for being attachable to the actuator housing 2 via a snap-in connection. The add-on housing 19 is attachable to the actuator housing 2 in such a way that the first and second switches 16a, 16b are triggerable by the first and second trigger members 12a, 12b of the metered dose inhaler 1. Upon attachment of the add-on housing 19 the electronic unit 14 is positioned at the rear side 4 of the actuator housing 2 in proximity to the first open end 5 of the actuator housing 2.

The electronic unit 14 may comprise an additional switch which is formed as a third switch 16c. The third switch 16c may be triggerable by the actuation housing 2 upon attachment of the add-on housing 19 to the actuator housing 2.

In order to allow detachment of the add-on housing 19 together with the electronic unit 14 fixed thereto, the add-on housing 19 comprises a release mechanism (not shown). The release mechanism is configured to release the snap-in members 21 from the corresponding openings on the rear side 4 of the actuator housing 2 upon manual actuation. This release of the snap-in members 21 allows detachment of the add-on housing 19 together with the electronic unit 14 from the actuator housing 2 independently from the triggering unit 14 which stays within the actuator housing 2. The add-on housing 19 together with the electronic unit 14 is configured to be re-usable in combination with another metered dose inhaler 1, preferably with a metered dose inhaler 1 as described above.

In the following, the assembly as well as the function of the metered dose inhaler 1 shall be explained with reference to Figures 1 and 2.

The metered dose inhaler 1 may be assembled by a patient or a specialist such as for example a physician or a pharmacist as follows:
The add-on device 13 is moved along an attachment direction B towards the actuator housing 2 such that the snap-in members 21 of the add-on device 13 are inserted into corresponding receiving members formed as openings at the rear side 4 of the actuator housing 2 thereby establishing a snap-in connection between the add-on housing 19 and the actuator housing 2. By attaching the add-on device 13 to the actuator housing 2 the first and second switches 16a, 16b of the electronic unit 14 of the add-on device 13 are positioned relatively to the first and second trigger members 16a, 16b of the triggering unit 11 in such a way that the first and second switches 16a, 16b are triggerable by the first and second trigger members 12a, 12b of the metered dose inhaler 1.

When attaching the add-on device to the actuator housing 2, the establishing of a snap-in connection may lead to a trigger event in such a way that the actuator housing 2 triggers the third switch 16c of the electronic unit 14. This triggering event might indicate that the electronic unit was attached to the actuator housing 2. In case the container 6 or the actuator housing 2 comprises a RFID tag the electronic unit 14 may subsequently read out information stored on the RFID tag. Alternatively, if an identification mark such as a QR-code, RFID Tag or a serial number is located on the container 6 or the actuator housing 2 the electronic unit 14 or the mobile device may request the patient or the specialist to enter the serial number in a mobile device such as a smartphone or to read the QR-code or the RFID tag with such a smartphone upon establishing of the snap-in connection between the add-on housing 19 and the actuator housing 2. This requires a wireless connection between the electronic unit 14 and the mobile device. However, electronic units configured to establish such a wireless connection with a mobile device are well known.

Moreover, in case a mechanical dose counter (not shown) is used in combination with the present metered dose inhaler 1 the electronic unit 14 may request the patient or the specialist to read out the dispensed or remaining doses counted by the mechanical dose counter and enter them into the mobile device or to use the mobile device to read them upon attachment of the add-on housing 19 to the actuator housing 2.

Having properly assembled the metered dose inhaler 1 as explained above, the function of the metered dose inhaler 1 is explained in the following:
Upon manual actuation of the container 6, such as pressing the container 6 in longitudinal direction A towards the bottom portion 9 of the actuator housing 2, the container 6 moves in the longitudinal direction A from a rest position as shown in Figure 1 to an activation position. During this movement, the container 6 interacts with the first and second trigger members 12a, 12b. The first and second trigger members 12a, 12b are moved laterally to the longitudinal direction A and at least partially deform the rear side 4 of the actuator housing 2 in an elastic way in order to trigger the first and second switches 16a, 16b of the electronic unit 14. Subsequently the counting circuit 17 receives signals from the first and second switches 16a, 16b indicating first and second times when the first and second switches 16a, 16b are triggered by the first and second trigger members 12a, 12b. Upon receipt and evaluation of the signals by a processing unit (not shown), the dispensed doses or doses remaining in the container 6 are counted by the counting circuit. The detailed function of a metered dose inhaler as described above, is disclosed in WO 2016/030844 A1.

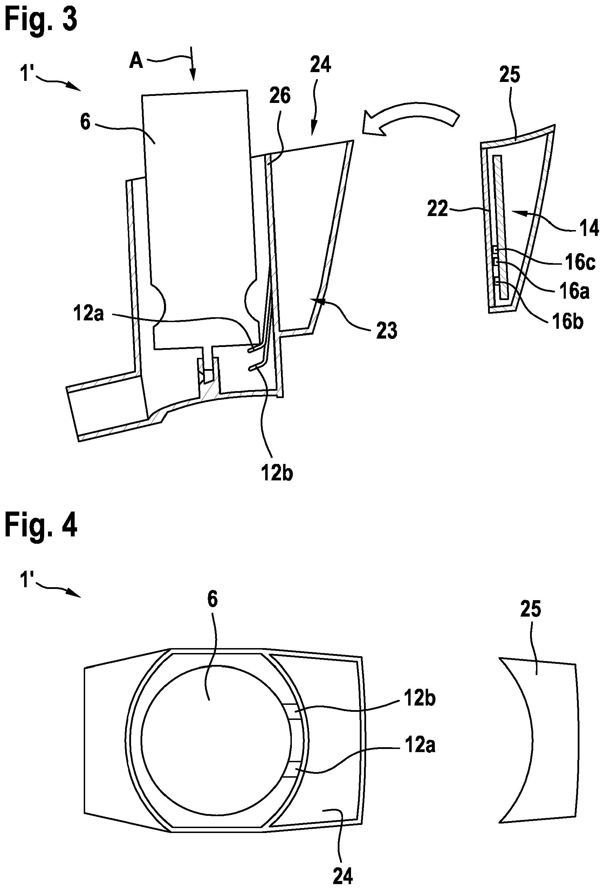

In Figures 3 and 4, a second embodiment of the metered dose inhaler 1 is shown. The metered dose inhaler 1' differs from the one shown in the Figures 1 and 2 by the carrier 18 which is formed as a frame 22 to which the electronic unit 14 is fixed.

The metered dose inhaler 1' further differs from the one shown in Figures 1 and 2 by a receiving portion 23 which is formed by the rear side 4 of the actuator housing 2 in proximity to the first open end 5 of the actuator housing 2. The receiving portion 23 comprises a top opening 24 through which the frame 22 may be inserted into the receiving portion 23. The receiving portion 23 is sized and arranged to receive the frame 22 together with the electronic unit 14 such that the electronic unit 14 is positioned between the rear side 4 of the actuator housing 2 and the container 6. The frame 22 has a top cover 25 on its top which covers the top opening 24 of the receiving portion 23 when the frame 22 is inserted into the receiving portion 23.

The metered dose inhaler 1' further differs from the one shown in Figure 1 and 2 by the actuator housing 2 which comprises a middle wall 26 positioned between the first open end 5 of the actuator housing 2 and the receiving portion 23. The first and second trigger members 12a, 12b extend from the middle wall 26 towards the second open end 10 of the actuator housing 2. The first and second trigger members 12a, 12b are configured to at least partially deform the middle wall 26 of the actuator housing 2 in order to trigger the first and second switches 16a, 16b.

The frame 22 is connected to the actuator housing 2 via a snap-in connection, wherein the attachment members of the frame 22 are configured as snap-in members (not shown). Preferably, the receiving portion 23 comprises guiding members (not shown) which guide the frame 22 when being inserted through the top opening 24 into the receiving portion. The snap-in members may be formed as protrusions which engage with corresponding receiving members formed as openings (not shown) positioned in the receiving portion 23.

In the following, the assembly as well as the function of the metered dose inhaler 1' shall be explained with reference to Figures 3 and 4.

As described above, the metered dose inhaler 1' is assembled by inserting the frame 22 together with the electronic unit 14 into the receiving portion 23 from above. Preferably, the frame 22 slides into guiding elements positioned in the receiving portion 23 such that the frame 22 being inserted into the receiving portion slides along the longitudinal direction A into the receiving portion 23. In order to fix the frame 22 in the receiving portion, the frame 22 may comprise protrusions which engage with corresponding holes within the receiving portion 23. The top cover 25 of the frame 22 close the top opening 24 of the receiving portion 23.

The function of the metered dose inhaler 1' is identical to the one described in connection with the metered dose inhaler 1 shown in Figures 1 and 2. The only difference is that upon interaction of the first and second trigger members 12a, 12b with the container 6 moving from a rest position to an activation position the trigger members 12a, 12b partially deform the middle of wall 26 instead of the rear side 4 of the actuator housing 2 as described in combination with the metered dose inhaler shown in Figures 1 and 2.

### REFERENCE NUMERALS

- 1, 1': metered dose inhaler (MDI)
- 2: actuator housing
- 3: front side (housing)
- 4: rear side (housing)
- 5: first open end (housing)
- 6: aerosol container
- 7: valve
- 8: valve end (container)
- (A): longitudinal direction
- 9: bottom portion (housing)
- 10: second open end (housing)
- 11: triggering unit
- 12a, 12b: first/second trigger member
- 13: add-on device
- 14: electronic unit
- 15: substrate
- 16a, 16b,: 16c first/second/third switch
- 17: counting circuit
- 18: carrier
- 19: add-on housing (carrier)
- (B): attachment direction
- 20: opening (add-on housing)
- 21: snap-in members
- 22: frame (carrier)
- 23: receiving portion (housing)
- 24: top opening
- 25: top cover
- 26: middle wall

## Claims

1. Metered dose inhaler for dispensing aerosol doses comprising:
- an actuator housing (2) with
a front side (3) and a rear side (4) opposite the front side (3),
a first open end (5) sized and arranged to receive an aerosol container (6) with an activation valve (7) at a valve end (8) of the container (6), the container (6) being configured to move in a longitudinal direction (A) from a rest position to an activation position in which the valve (7) is depressed against a bottom portion (9) of the actuator housing (2) such that an aerosol dose is released and
a second open end (10) through which the released aerosol dose is dispensed, the second open end (10) being positioned on the front side (3) of the actuator housing (2) and sized and arranged to be coupled to the mouth or nasal cavities of a patient,
wherein the metered dose inhaler (1, 1') further comprises
- a triggering unit (11) having at least a first trigger member (12a) thereon, the triggering unit (11) being configured to interact with the container (6) when the container (6) moves in the longitudinal direction (A) from the rest position to the activation position, wherein the triggering unit (11) is fixed to or integrally formed with the actuator housing (2);
- an electronic unit (14) having a substrate (15) with at least a first switch (16a) thereon, the first switch (16a) being configured to interact with the first trigger member (12a) when the container (6) moves from the rest position to the activation position such that the first trigger member (12a) triggers the first switch (16a) when the container (6) reaches a first longitudinal position during movement of the container (6) from the rest position to the activation position,
- a counting circuit (17) which is configured to receive a signal from the first switch (16a) indicating at least a first time when the first switch (16a) is triggered by the first trigger member (12a), the counting circuit (17) being arranged on the substrate (15) of the electronic unit (14),
wherein the electronic unit (14) is detachable from the actuator housing (2) independently from the triggering unit (11),
wherein the electronic unit (14) is configured to be reusable in combination with another metered dose inhaler (1, 1') and,
that the metered dose inhaler (1, 1') comprises a carrier (18) for carrying and fixing the electronic unit (14) wherein the carrier (18) has attachment members (21) for being attached to the actuator housing (2),
**characterized in**
**that** the carrier (18) comprises a release mechanism configured to release the attachment members (21) upon manual actuation thereby allowing detachment of the carrier (18) together with the electronic unit (14) from the actuator housing (2).

2. Metered dose inhaler according to claim 1, **characterized in that** the electronic unit (14) is positioned at the rear side (4) of the actuator housing (2).

3. Metered dose inhaler according to claim 2, **characterized in that** the carrier (18) is formed as an add-on housing (19), having at least one opening (20) or one flexible area on one side facing the actuator housing (2) in order to allow interaction between the at least first trigger member (12a) and the at least first switch (16a).

4. Metered dose inhaler according to claim 3, **characterized in that** the add-on housing (19) is connected with the actuator housing (2) via a snap-in connection, wherein the attachment members are configured as snap-in members (21).

5. Metered dose inhaler according to any of the preceding claims, **characterized in that** the at least first trigger member (12a) extends from the rear side (4) of the actuator housing (2) towards the second open end (10) of the actuator housing (2), wherein the at least first trigger member (12a) is configured to at least partially deform the rear side (4) of the actuator housing (2) in order to trigger the first switch (16a).

6. Metered dose inhaler according to claim 3, **characterized in that** the carrier (18) is formed as a frame (22) to which the electronic unit (14) is fixed, wherein the rear side (4) of the actuator housing (2) forms a receiving portion (23) in proximity to the first open end (5) of the actuator housing (2), the receiving portion (23) being sized and arranged to receive the frame (22) together with the electronic unit (14) such that the electronic unit (14) is positioned between the rear side (4) of the actuator housing (2) and the container (6).

7. Metered dose inhaler according to claim 6, **characterized in that** the frame (22) is connected with the actuator housing (2) via a snap-in connection, wherein the attachment members are configured as snap-in members (21).

8. Metered dose inhaler according to claim 7, **characterized in that** the actuator housing (2) comprises a middle wall (26) positioned between the first open end (5) of the actuator housing (2) and the receiving portion (23), wherein the at least first trigger member (12a) extends from the middle wall (26) towards the second open end (10) of the actuator housing (2) and is configured to at least partially deform the middle wall (26) of the actuator housing (2) in order to trigger the first switch (16a).

9. Metered dose inhaler according to any of the preceding claims, **characterized in that** the attachment members (21) as well as the corresponding receiving members are configured that only matching attachment and receiving members (21) form a connection.

10. Metered dose inhaler according to any of the preceding claims, **characterized in that** the container (6) or the actuator housing (2) comprises an identification mark such as a RFID tag or a QR-code identifying for example the medicament contained in the container (6).

11. Metered dose inhaler according to any of the preceding claims, **characterized in that** the metered dose inhaler (1, 1') is operable with the electronic unit (14) being detached, wherein the electronic unit (14) is replaced by a dummy part.

12. Metered dose inhaler according to any of the preceding claims, **characterized in that** metered dose inhaler (1, 1') comprise a mechanical dose counter installed in the longitudinal direction (A) between the container (6) and the bottom portion (9) of the actuator housing (2).

13. Add-on device for being attached to an actuator housing of a metered dose inhaler (1, 1'), the add-on device (19) comprising :
- an electronic unit (14) having a substrate (15) with at least a first switch (16a) thereon, the first switch (16a) being configured to interact with a first trigger member (12a) of the metered dose inhaler (1, 1') when a container (6) received in an actuator housing (2) of the metered dose inhaler (1, 1') moves from a rest position to an activation position such that the first trigger member (12a) triggers the first switch (16a) when the container (6) reaches a first longitudinal position during movement of the container (6) from the rest position to the activation position and
- a counting circuit (17) that is configured to receive a signal from the first switch (16a) indicating at least a first time when the first switch (16a) is triggered by the first trigger member (12a), the counting circuit (17) being arranged on the substrate (15) of the electronic unit (14),
- a carrier (18) for carrying the electronic unit (14), wherein the carrier (18) comprises attachment members (21) for attaching the add-on device (19) to the actuator housing (2) of a metered dose inhaler (1, 1') in such a way that the first switch (16a) is triggerable by the first trigger member (12a) of the metered dose inhaler (1, 1'),
**characterized in**
**that** the carrier (18) comprises a release mechanism configured to release the attachment members (21) upon manual actuation thereby allowing detachment of the carrier (18) together with the electronic unit (14) from the actuator housing (2).

14. Add-on device according to claim 13, **characterized in that** the carrier (18) is formed as an add-on housing (19) having at least one opening (20) or one flexible area on a side of the add-on housing (19) configured to allow interaction between the at least first trigger member (12a) and the at least first switch (16a).

15. Add-on device according to claim 13, **characterized in that** the carrier (18) is formed as a frame (22) to which the electronic unit (14) is fixed, wherein the frame (22) is configured to be received in a receiving portion (23) formed by a rear side (4) of the actuator housing (2), such that the electronic unit (14) is positioned between the rear side (4) of the actuator housing (2) and the container (6).

## Patentansprüche

1. Dosierinhalator zum Abgeben von Aerosoldosen umfassend:
- ein Aktuatorgehäuse (2) mit
einer Vorderseite (3) und einer Rückseite (4), die gegenüber der Vorderseite (3) angeordnet ist,
einem ersten offenen Ende (5), das derart dimensioniert und angeordnet ist, um einen Aerosol-Behälter (6) mit einem Betätigungsventil (7) an einem Ventilende (8) des Behälters (6) aufzunehmen, wobei der Behälter (6) ausgebildet ist, sich in einer Längsrichtung (A) von einer Ruheposition in eine Betätigungsposition zu bewegen, in der das Ventil (7) gegen einen Bodenteil (9) des Aktuatorgehäuses (2) gedrückt wird, so dass eine Aerosoldose freigesetzt wird, und einem zweiten offenen Ende (10), durch das die freigesetzte Aerosoldose abgegeben wird, wobei das zweite offene Ende (10) an der Vorderseite (3) des Aktuatorgehäuses (2) positioniert ist und derart dimensioniert und angeordnet ist, um mit dem Mund oder den Nasenhöhlen eines Patentens gekoppelt zu werden,
wobei der Dosierinhalator (1, 1') weiter umfasst
- eine Auslöseeinheit (11), die mindestens ein erstes Auslöseelement (12a) darauf hat, wobei die Auslöseeinheit (11) ausgebildet ist, mit dem Behälter (6) zu interagieren, wenn sich der Behälter (6) in der Längsrichtung (A) von der Ruheposition in die Betätigungsposition bewegt, wobei die Auslöseeinheit (11) an dem Aktuatorgehäuse (2) befestigt oder einstückig mit diesem geformt ist;
- eine Elektronikeinheit (14), die ein Trägermaterial (15) mit mindestens einem ersten Schalter (16a) darauf umfasst, wobei der erste Schalter (16a) ausgebildet ist, mit dem ersten Betätigungselement (12a) zu interagieren, wenn sich der Behälter (6) von der Ruheposition in die Betätigungsposition bewegt, so dass das erste Betätigungselement (12a) den ersten Schalter (16a) betätigt, wenn der Behälter (6) eine erste Längsposition während der Bewegung des Behälters (6) von der Ruheposition in die Betätigungsposition erreicht,
- einen Zählschaltkreis (17) der ausgebildet ist, ein Signal von dem ersten Schalter (16a) zu empfangen, das mindestens einen ersten Zeitpunkt angibt, wenn der erste Schalter (16a) von dem ersten Betätigungselement (12a) betätigt wird, wobei der Zählschaltkreis (17) auf dem Trägermaterial (15) der Elektronikeinheit (14) angeordnet ist,
wobei die Elektronikeinheit (14) von dem Aktuatorgehäuse (2) unabhängig von der Betätigungseinheit (11) lösbar ist, wobei die Elektronikeinheit (14) ausgebildet ist, in Kombination mit einem anderen Dosierinhalator (1, 1') wiederverwendbar zu sein und,
dass der Dosierinhalator (1, 1') einen Träger (18) zum Tragen und Fixieren der Elektronikeinheit (14) umfasst, wobei der Träger (18) Befestigungselemente (21) umfasst, um an dem Aktuatorgehäuse (2) befestigt zu werden,
**dadurch gekennzeichnet,**
**dass** der Träger (18) einen Lösemechanismus umfasst, der ausgebildet ist, bei manueller Betätigung die Befestigungselemente (21) freizugeben, wodurch ein Ablösen des Trägers (18) zusammen mit der Elektronikeinheit (14) von dem Aktuatorgehäuse (2) ermöglicht wird.

2. Dosierinhalator nach Anspruch 1, **dadurch gekennzeichnet, dass** die Elektronikeinheit (14) an der Rückseite (4) des Aktuatorgehäuses (2) positioniert ist.

3. Dosierinhalator nach Anspruch 2, **dadurch gekennzeichnet, dass** der Träger (18) als ein Erweiterungsgehäuse (19) geformt ist, das mindestens eine Öffnung (20) oder einen flexiblen Bereich auf einer Seite hat, die dem Aktuatorgehäuse (2) zugewandt ist, um ein Interagieren zwischen dem mindestens einen Betätigungselement (12a) und dem mindestens einen Schalter (16a) zu ermöglichen.

4. Dosierinhalator nach Anspruch 3, **dadurch gekennzeichnet, dass** das Erweiterungsgehäuse (19) mit dem Aktuatorgehäuse (2) über eine Schnappverbindung verbunden ist, wobei die Befestigungselemente als Schnappelemente (21) ausgebildet sind.

5. Dosierinhalator nach einem der vorhergehenden Ansprüche, **dadurch gekennzeichnet, dass** sich das mindestens eine Betätigungselement (12a) von der Rückseite (4) des Aktuatorgehäuses (2) in Richtung des zweiten offenen Endes (10) des Aktuatorgehäuses (2) erstreckt, wobei das mindestens erste Betätigungselement (12a) ausgebildet ist, die Rückseite (4) des Aktuatorgehäuses (2) zumindest teilweise zu verformen, um den ersten Schalter (16a) zu betätigen.

6. Dosierinhalator nach Anspruch 3, **dadurch gekennzeichnet, dass** der Träger (18) als Rahmen (22) geformt ist, an dem die Elektronikeinheit (14) fixiert ist, wobei die Rückseite (4) des Aktuatorgehäuses (2) ein Aufnahmeteil (23) nahe dem ersten offenen Ende (5) des Aktuatorgehäuses (2) bildet, wobei der Aufnahmeteil (23) derart dimensioniert und angeordnet ist, um den Rahmen (22) zusammen mit der Elektronikeinheit (14) aufzunehmen, so dass die Elektronikeinheit (14) zwischen der Rückseite (4) des Aktuatorgehäuses (2) und dem Behälter (6) positioniert ist.

7. Dosierinhalator nach Anspruch 6, **dadurch gekennzeichnet, dass** der Rahmen (22) mit dem Aktuatorgehäuse (2) mittels einer Schnappverbindung verbunden ist, wobei die Befestigungselemente als Schnappelemente (21) ausgebildet sind.

8. Dosierinhalator nach Anspruch 7, **dadurch gekennzeichnet, dass** das Aktuatorgehäuse (2) eine Mittenwand (26) umfasst, die zwischen dem ersten offenen Ende (5) des Aktuatorgehäuses (2) und dem Aufnahmeteil (23) positioniert ist, wobei sich das mindestens erste Betätigungselement (12a) von der Mittenwand (26) in Richtung des zweiten offenen Endes (10) des Aktuatorgehäuses (2) erstreckt und ausgebildet ist, mindestens teilweise die Mittenwand (26) des Aktuatorgehäuses (2) zu verformen, um den ersten Schalter (16a) zu betätigen.

9. Dosierinhalator nach einem der vorhergehenden Ansprüche, **dadurch gekennzeichnet, dass** die Befestigungselemente (21) und die entsprechenden Aufnahmeelemente ausgebildet sind, dass nur zueinander passende Befestigungs- und Aufnahmeelemente (21) eine Verbindung bilden.

10. Dosierinhalator nach einem der vorhergehenden Ansprüche, **dadurch gekennzeichnet, dass** der Behälter (6) oder das Aktuatorgehäuse (2) ein Identifikationszeichen, wie beispielsweise ein RFID-Tag oder einen QR-Code, umfasst, der zum Beispiel das Medikament bezeichnet, das in dem Behälter (6) enthalten ist.

11. Dosierinhalator nach einem der vorhergehenden Ansprüche, **dadurch gekennzeichnet, dass** der Dosierhinalator (1, 1') mit abgelöster Elektronikeinheit (14) betriebsbereit ist, wobei die Elektronikeinheit (10) durch ein Dummy-Teil ersetzt wird.

12. Dosierinhalator nach einem der vorhergehenden Ansprüche, **dadurch gekennzeichnet, dass** der Dosierinhalator (1, 1') einen mechanischen Dosiszähler umfasst, der in Längsrichtung (A) zwischen dem Behälter (6) und dem Bodenteil (9) des Aktuatorgehäuses (2) installiert ist.

13. Erweiterungsvorrichtung um an einem Aktuatorgehäuse eines Dosierinhalators (1, 1') befestigt zu werden, wobei die Erweiterungsvorrichtung (19) umfasst:
- eine Elektronikeinheit (14), die ein Trägermaterial (15) mit mindestens einem ersten Schalter (16a) darauf hat, wobei der erste Schalter (16a) ausgebildet ist, mit einem ersten Auslöseelement (12a) des Dosierinhalators (1, 1') zu interagieren, wenn sich ein Behälter (6), der in einem Aktuatorgehäuse (2) des Dosierinhalators (1 1') aufgenommen wird, von einer Ruheposition in eine Betätigungsposition bewegt, so dass das erste Betätigungselement (12a) den ersten Schalter (16a) betätigt, wenn der Behälter (6) eine erste Längsposition während der Bewegung des Behälters (6) von der Ruheposition in die Betätigungsposition erreicht, und
- einen Zählschaltkreis (17), der ausgebildet ist, ein Signal von dem ersten Schalter (16a) zu empfangen, das mindestens einen ersten Zeitpunkt angibt, wenn der erste Schalter (16a) von dem ersten Betätigungselement (12a) betätigt wird, wobei der Zählschaltkreis (17) auf dem Trägermaterial (15) der Elektronikeinheit (14) angeordnet ist,
- einen Träger (18) zum Tragen der Elektronikeinheit (14),
wobei der Träger (18) Befestigungselemente (21) zum derartigen Befestigen der Erweiterungsvorrichtung (19) an dem Aktuatorgehäuse (2) des Dosierinhalators (1, 1') umfasst, dass der erste Schalter (16a) von dem ersten Betätigungselement (12a) des Dosierinhalators (1, 1') betätigbar ist,
**dadurch gekennzeichnet,**
**dass** der Träger (18) einen Lösemechanismus umfasst, der ausgebildet ist, bei manueller Betätigung die Befestigungselemente (21) freizugeben, wodurch ein Ablösen des Trägers (18) zusammen mit der Elektronikeinheit (14) von dem Aktuatorgehäuse (2) ermöglicht wird.

14. Erweiterungsvorrichtung nach Anspruch 13, **dadurch gekennzeichnet, dass** der Träger (18) als Erweiterungsgehäuse (19) ausgebildet ist, das mindestens eine Öffnung (20) oder einen flexiblen Bereich auf einer Seite des Erweiterungsgehäuses (19) hat, die/der ausgebildet ist, ein Interagieren zwischen dem mindestens ersten Betätigungselement (12a) und dem mindestens ersten Schalter (16a) zu ermöglichen.

15. Erweiterungsvorrichtung nach Anspruch 13, **dadurch gekennzeichnet, dass** der Träger (18) als Rahmen (22) geformt ist, an dem der Elektronikschaltkreis (14) fixiert ist, wobei der Rahmen (22) ausgebildet ist, in einem Aufnahmeteil (23) aufgenommen zu werden, der von einer Rückseite (4) des Aktuatorgehäuses (2) gebildet wird, so dass die Elektronikeinheit (14) zwischen der Rückseite (4) des Aktuatorgehäuses (2) und dem Behälter (6) positioniert ist.

## Revendications

1. Inhalateur de dose mesurée pour distribuer des doses d'aérosol, comprenant :
- un logement d'actionneur (2) comprenant une face avant (3) et une face arrière (4) opposée à la face avant (3),
une première extrémité ouverte (5) dimensionnée et agencée pour recevoir un contenant d'aérosol (6) avec une valve d'activation (7) à une extrémité de valve (8) du contenant (6), le contenant (6) étant configuré pour se déplacer dans une direction longitudinale (A) d'une position de repos à une position d'activation dans laquelle la valve (7) est enfoncée contre une partie inférieure (9) du logement d'actionneur (2) de telle façon qu'une dose d'aérosol soit libérée et
une seconde extrémité ouverte (10) à travers laquelle la dose d'aérosol libérée est distribuée, la seconde extrémité ouverte (10) étant positionnée sur la face avant (3) du logement d'actionneur (2) et dimensionnée et agencée pour être couplée à la bouche ou aux cavités nasales d'un patient,
dans lequel l'inhalateur de dose mesurée (1, 1') comprend en outre
- une unité de déclenchement (11) ayant au moins un premier membre déclencheur (12a) dessus, l'unité de déclenchement (11) étant configurée pour interagir avec le contenant (6) lorsque le contenant (6) se déplace dans la direction longitudinale (A) de la position de repos à la position d'activation, dans lequel l'unité de déclenchement (11) est fixée au, ou formée intégralement avec le, logement d'actionneur (2) ;
- une unité électronique (14) ayant un substrat (15) avec au moins un premier bouton (16a) dessus, le premier bouton (16a) étant configuré pour interagir avec le premier membre déclencheur (12a) lorsque le contenant (6) se déplace de la position de repos à la position d'activation de telle façon que le premier membre déclencheur (12a) déclenche le premier bouton (16a) lorsque le contenant (6) atteint une première direction longitudinale pendant le mouvement du contenant (6) de la position de repos à la position d'activation,
- un circuit de comptage (17) qui est configuré pour recevoir un signal du premier bouton (16a) indiquant au moins une première fois où le premier bouton (16a) est déclenché par le premier membre déclencheur (12a), le circuit de comptage (17) étant agencé sur le substrat (15) de l'unité électronique (14),
dans lequel l'unité électronique (14) peut être séparée du logement d'actionneur (2) indépendamment de l'unité de déclenchement (11),
dans lequel l'unité électronique (14) est configurée pour être réutilisable en combinaison avec un autre inhalateur de dose mesurée (1, 1'), et
que l'inhalateur de dose mesurée (1, 1') comprend un support (18) pour supporter et fixer l'unité électronique (14), dans lequel le support (18) présente des membres de fixation (21) pour être fixé au logement d'actionneur (2),
**caractérisé en ce que**
le support (18) comprend un mécanisme de libération configuré pour libérer les membres de fixation (21) lors de l'actionnement manuel, permettant ainsi la séparation du support (18) conjointement avec l'unité électronique (14), du logement d'actionneur (2).

2. Inhalateur de dose mesurée selon la revendication 1, **caractérisé en ce que** l'unité électronique (14) est positionnée à l'arrière (4) du logement d'actionneur (2) .

3. Inhalateur de dose mesurée selon la revendication 2, **caractérisé en ce que** le support (18) est formé en tant qu'un logement additionnel (19) ayant au moins une ouverture (20) ou une zone flexible sur un côté face au logement d'actionneur (2) afin de permettre l'interaction entre l'au moins premier membre déclencheur (12a) et l'au moins premier bouton (16a).

4. Inhalateur de dose mesurée selon la revendication 3, **caractérisé en ce que** le logement additionnel (19) est relié au logement d'actionneur (2) par une connexion par encliquetage, dans lequel les membres de fixation sont configurés comme des membres d'encliquetage (21).

5. Inhalateur de dose mesurée selon l'une quelconque des revendications précédentes, **caractérisé en ce que** l'au moins premier membre déclencheur (12a) s'étend de l'arrière (4) du logement d'actionneur (2) vers la seconde extrémité ouverte (10) du logement d'actionneur (2), dans lequel l'au moins premier membre déclencheur (12a) est configuré pour déformer au moins partiellement l'arrière (4) du logement d'actionneur (2) afin de déclencher le premier bouton (16a) .

6. Inhalateur de dose mesurée selon la revendication 3, **caractérisé en ce que** le support (18) est formé en tant qu'un cadre (22) auquel l'unité électronique (14) est fixée, dans lequel l'arrière (4) du logement d'actionneur (2) forme une partie réceptrice (23) à proximité de la première extrémité ouverte (5) du logement d'actionneur (2), la partie réceptrice (23) étant dimensionnée et agencée pour recevoir le cadre (22) conjointement avec l'unité électronique (14) de façon à ce que l'unité électronique (14) soit positionnée entre l'arrière (4) du logement d'actionneur (2) et le contenant (6).

7. Inhalateur de dose mesurée selon la revendication 6, **caractérisé en ce que** le cadre (22) est relié au logement d'actionneur (2) par une connexion par encliquetage, dans lequel les membres de fixation sont configurés comme des membres d'encliquetage (21).

8. Inhalateur de dose mesurée selon la revendication 7, **caractérisé en ce que** le logement d'actionneur (2) comprend une paroi centrale (26) positionnée entre la première extrémité ouverte (5) du logement d'actionneur (2) et la partie réceptrice (23), dans lequel l'au moins premier membre déclencheur (12a) s'étend de la paroi centrale (26) vers la seconde extrémité ouverte (10) du logement d'actionneur (2) et est configuré pour déformer au moins partiellement la paroi centrale (26) du logement d'actionneur (2) afin de déclencher le premier bouton (16a).

9. Inhalateur de dose mesurée selon l'une quelconque des revendications précédentes, **caractérisé en ce que** les membres de fixation (21) ainsi que les membres de réception correspondants sont configurés pour que seuls des membres de fixation et des membres de réception (21) correspondants forment une connexion.

10. Inhalateur de dose mesurée selon l'une quelconque des revendications précédentes, **caractérisé en ce que** le contenant (6) ou le logement d'actionneur (2) comprend un marquage d'identification tel qu'une étiquette RFID ou un QR code identifiant par exemple le médicament contenu dans le contenant (6).

11. Inhalateur de dose mesurée selon l'une quelconque des revendications précédentes, **caractérisé en ce que** l'inhalateur de dose mesurée (1, 1') peut être actionné avec l'unité électronique (14) détachée, dans lequel l'unité électronique (14) est remplacée par une pièce factice.

12. Inhalateur de dose mesurée selon l'une quelconque des revendications précédentes, **caractérisé en ce que** l'inhalateur de dose mesurée (1, 1') comprend un compteur de doses mécanique monté dans la direction longitudinale (A) entre le contenant (6) et la partie inférieure (9) du logement d'actionneur (2).

13. Dispositif additionnel destiné à être fixé à un logement d'actionneur d'un inhalateur de dose mesurée (1, 1'), le dispositif additionnel (19) comprenant :
- une unité électronique (14) ayant un substrat (15) avec au moins un premier bouton (16a) dessus, le premier bouton (16a) étant configuré pour interagir avec un premier membre déclencheur (12a) de l'inhalateur de dose mesurée (1, 1') lorsqu'un contenant (6) reçu dans un logement d'actionneur (2) de l'inhalateur de dose mesurée (1, 1') se déplace d'une position de repos à une position d'activation de telle façon que le premier membre déclencheur (12a) déclenche le premier bouton (16a) lorsque le contenant (6) atteint une première direction longitudinale pendant le mouvement du contenant (6) de la position de repos à la position d'activation,
- un circuit de comptage (17) qui est configuré pour recevoir un signal du premier bouton (16a) indiquant au moins une première fois où le premier bouton (16a) est déclenché par le premier membre déclencheur (12a), le circuit de comptage (17) étant agencé sur le substrat (15) de l'unité électronique (14),
- un support (18) pour supporter l'unité électronique (14), dans lequel le support (18) présente des membres de fixation (21) pour fixer le dispositif additionnel (19) au logement d'actionneur (2) d'un inhalateur de dose mesurée (1, 1') de telle façon que le premier bouton (16a) puisse être déclenché par le premier membre déclencheur (12a) de l'inhalateur de dose mesurée (1, 1'),
**caractérisé en ce que**
le support (18) comprend un mécanisme de libération configuré pour libérer les membres de fixation (21) lors de l'actionnement manuel, permettant ainsi la séparation du support (18) conjointement avec l'unité électronique (14), du logement d'actionneur (2).

14. Dispositif additionnel selon la revendication 13, **caractérisé en ce que** le support (18) est formé en tant qu'un dispositif additionnel (19) ayant au moins une ouverture (20) ou une zone flexible sur un côté du dispositif additionnel (19) configurée pour permettre l'interaction entre l'au moins premier membre déclencheur (12a) et l'au moins premier bouton (16a).

15. Dispositif additionnel selon la revendication 13, **caractérisé en ce que** le support (18) est formé en tant qu'un cadre (22) auquel l'unité électronique (14) est fixée, dans lequel le cadre (22) est configuré pour être reçu dans une partie réceptrice (23) formée par un arrière (4) du logement d'actionneur (2), de façon à ce que l'unité électronique (14) soit positionnée entre l'arrière (4) du logement d'actionneur (2) et le contenant (6).
